Europäisches Patentamt

**European Patent Office**

Office européen des brevets

(11) Numéro de publication: **0 011 019**
**A1**

## DEMANDE DE BREVET EUROPEEN

(21) Numéro de dépôt: 79400782.3

(22) Date de dépôt: 23.10.79

(51) Int. Cl.³: **A 61 N 1/40**

(30) Priorité: 30.10.78 FR 7830754

(43) Date de publication de la demande: 14.05.80
Bulletin 80/10

(84) Etats contractants désignés: AT BE CH DE GB IT LU NL SE

(71) Demandeur: Astier, Jean Albert Lucien, 99, rue Gilbert Rousset, F-92000 Asnieres (FR)

(72) Inventeur: Astier, Jean Albert Lucien, 99, rue Gilbert Rousset, F-92000 Asnieres (FR)

(74) Mandataire: Corre, Jacques et al, Cabinet Regimbeau 26, Avenue Kléber, F-75116 Paris (FR)

(54) **Appareil de traitement biologique électromagnétique à polarisation circulaire.**

(57) La présente invention concerne un appareil de traitement biologique par rayonnement électromagnétique.

Un oscillateur 1 produit une fréquence 27,12 MHz qui est appliqué à un amplificateur accordé modulable 2, recevant d'un générateur de modulation 3 un signal de modulation en tout ou rien, de fréquence de récurrence et de facteur de forme réglable. La sortie sortie de l'amplificateur 2 est appliquée directement à l'amplificateur de puissance 4 commandant un premier cadre 5. Un second cadre 8, situé dans un plan perpendiculaire à celui du premier, reçoit la sortie d'un autre amplificateur de puissance 7, qui provient de l'amplificateur accordé 2 à travers un déphaseur 6 produisant un déphasage de 90° à la fréquence porteuse de 27,12 MHz.

Application aux traitements biologiques.

## Appareil de traitement biologique électromagnétique à polarisation circulaire

La présente invention concerne les appareils de traitement biologique par rayonnement électromagnétique.

On connaît déjà des appareils de ce genre qui comportent un radiateur électromagnétique, tel qu'un cadre, et un moyen pour exciter ce radiateur par un signal électrique haute fréquence de façon qu'il émette un champ électromagnétique correspondant. La plupart du temps, le radiateur n'est pas excité par un signal électrique haute fréquence permanent, mais par un signal haute fréquence modulée. La modulation s'effectue en tout ou rien, en général avec une fréquence de récurrence et un facteur de forme réglable. Le facteur de forme est choisi de façon que la durée de l'impulsion haute fréquence émise soit brève par rapport à la période de récurrence de ces impulsions.

Utilisés pour des traitements biologiques, sur l'animal et/ou sur l'homme, ces appareils se sont avérés avoir des effets bénéfiques sur l'activité cellulaire. A l'heure actuelle, ces effets sont loin d'être complètement expliqués. Ils semblent cependant se manifester au niveau du potentiel de membrane des cellules, et du cytosquelette excités d'une manière encore assez mal connue par le rayonnement électromagnétique.

La difficulté pratique vient du fait que les rayonnements électromagnétiques se propagent assez mal, et assez peu profondément dans les structures biologiques. Il en résulte un mauvais couplage de ces cellules avec le champ électromagnétique appliqué. Or ce couplage est à l'évidence nécessaire pour une bonne efficacité des traitements biologiques par onde électromagnétique.

La présente invention vient améliorer cette situation.

De façon très surprenante, il a été observé qu'en appliquant à des cellules un rayonnement électromagnétique haute fréquence polarisée circulairement, au lieu du rayonnement électromagnétique classique à polarisation linéaire, le transfert de l'énergie du rayonnement dans les cellules est sensiblement amélioré.

Ainsi, la présente invention prévoit un appareil de traitement biologique du type défini plus haut, dans lequel le champ électromagnétique est à polarisation circulaire.

Dans un mode de réalisation préférentiel, l'appareil comporte deux radiateurs électromagnétiques disposés pour rayonner à angle droit l'un de l'autre, et ces deux radiateurs sont excités par deux signaux déphasés de 90° en haute fréquence l'un par rapport à l'autre.

La plupart du temps, l'appareil utilisé selon la présente invention comporte comme moyen d'excitation un oscillateur dont la fréquence est liée à la fréquence porteuse, un préamplificateur accordé, comportant éventuellement une multiplication et une division de fréquence, et deux amplificateurs de puissance recevant tous deux la sortie du préamplificateur accordé, mais avec un déphasage de 90° en haute fréquence l'un par rapport à l'autre. Bien que ce déphasage à 90° puisse être réparti sous forme de deux déphasages de 45° en sens opposé, sur les deux voies qui mènent du préamplificateur aux amplificateurs de puissance, il est actuellement considéré comme préférable et plus simple de relier directement l'un des amplificateurs de puissance à la sortie du préamplificateur accordé, tandis que l'autre amplificateur de puissance est relié à cette même sortie à travers un déphaseur de 90° en haute fréquence.

Dans la pratique, il est généralement convenable que les deux radiateurs soient deux cadres, de préférence du type monospire, disposés dans deux plans perpendiculaires.

Selon une autre caractéristique de la présente invention, le préamplificateur accordé est du type à commande de modulation, définie par un générateur de modulation. Avantageusement, la modulation est en tout ou rien, et le générateur de modulation est du type à fréquence de récurrence et/ou à facteur de forme variable. Comme dans la technique antérieure, il demeure avantageux que la durée effective des impulsions émises soit faible devant leur période de récurrence.

La présente invention sera mieux comprise à la lecture de la description détaillée qui va suivre, faite en référence aux dessins annexés, donnés uniquement à titre d'exemple non limitatif, et sur lesquels :

- la figure 1 illustre un appareil de traitement biologique par rayonnement électromagnétique selon la technique antérieure ; et

- la figure 2 illustre un appareil de traitement biologique par onde électromagnétique selon la présente invention.

4 0011019

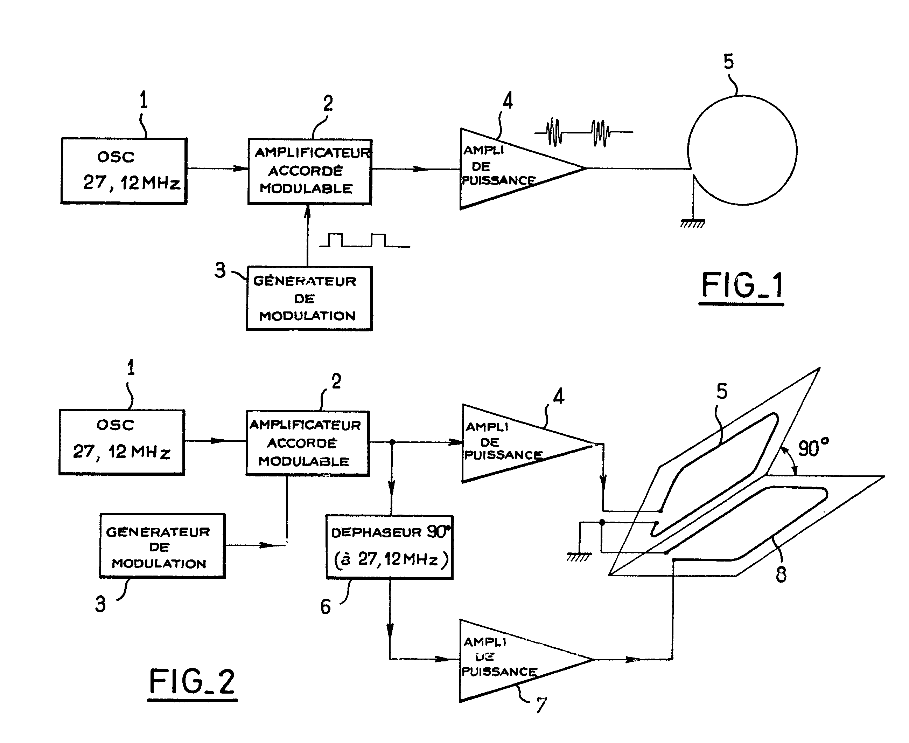

Sur la figure 1, un oscillateur à 27,12 MHz, désigné par la référence numérique 1, alimente un amplificateur accordé modulable 2. L'amplificateur accordé modulable 2 reçoit également d'un générateur de modulation 3 un signal de commande constitué d'impulsions à fréquence de récurrence et facteur de forme variable. La fréquence de récurrence peut par exemple être choisie parmi les valeurs suivantes :

de 50 à 1 000 Hz

De son côté, la durée de chaque impulsion de haute fréquence émise peut prendre les valeurs suivantes :

de 10 à 100 microsecondes.

La sortie de l'amplificateur accordé modulable 2, ainsi modulée d'après la sortie du générateur 3, attaque un amplificateur de puissance 4, relié à son tour à l'élément radiateur 5, qui prend ici la forme d'un cadre à spire unique.

On décrira maintenant la présente invention en référence à la figure 2, où les éléments qui sont semblables à ceux de la figure 1, portent les mêmes références.

Selon l'invention, la sortie de l'amplificateur accordé attaque comme précédemment l'amplificateur de puissance 4, qui est connecté à un premier cadre 5.

La sortie de l'amplificateur accordé modulable 2 est également appliquée à un déphaseur 6 produisant un déphasage de 90° à la fréquence de 27,12 MHz. La sortie du déphaseur 6 est à son tour appliquée à un second amplificateur de puissance 7, qui alimente un second cadre unispire 8, situé dans un plan perpendiculaire au plan dans lequel se trouve le premier cadre 5.

L'homme de l'art appréciera que de cette façon, on obtient un champ électromagnétique à polarisation circulaire si l'on place un organe biologique dans l'angle d'ouverture des deux plans, et entre les deux cadres 5 et 8.

La meilleure absorption du champ électromagnétique produit selon l'invention a pu être mise en évidence par les expérimentations suivantes, effectuées in vitro : en moins de deux minutes, des cellules 3T3 de souris s'arrondissent et deviennent beaucoup plus réfringentes, ce qui est caractéristique d'un réaménagement du cytosquelette.

REVENDICATIONS

1 - Appareil de traitement biologique, du type comportant un radiateur électromagnétique, tel qu'un cadre, et un moyen pour exciter ce radiateur par un signal électrique haute fréquence de façon qu'il émette un champ électromagnétique correspondant, caractérisé par le fait que ce champ électromagnétique est à polarisation circulaire.

2 - Appareil de traitement biologique selon la revendication 1, caractérisé par le fait qu'il comporte deux radiateurs disposés pour rayonner à angle droit l'un de l'autre, et que les deux radiateurs sont excités par deux signaux déphasés de 90° en haute fréquence l'un par rapport à l'autre.

3 - Appareil de traitement biologique selon la revendication 2, caractérisé par le fait que le moyen d'excitation comporte un oscillateur de fréquence lié à la fréquence porteuse, un préamplificateur accordé, et deux amplificateurs de puissance recevant tous deux la sortie du préamplificateur accordé, mais avec un déphasage de 90° en haute fréquence l'un par rapport à l'autre.

4 - Appareil de traitement biologique selon la revendication 3, caractérisé par le fait que l'un des amplificateurs de puissance est relié directement à la sortie du préamplificateur accordé, l'autre à cette même sortie à travers un déphaseur de 90° en haute fréquence.

5 - Appareil de traitement biologique selon l'une des revendications 3 et 4, caractérisé par le fait que le préamplificateur accordé est du type à commande de modulation, définie par un générateur de modulation.

6 - Appareil de traitement biologique selon la revendication 5, caractérisé par le fait que la modulation est en tout ou rien, et le générateur de modulation du type à fréquence de récurrence et/ou à facteur de forme variable.

7 - Appareil de traitement biologique selon l'une des revendications 1 à 5, caractérisé par le fait que les deux radiateurs sont deux cadres disposés dans deux plans perpendiculaires.

8 - Appareil de traitement biologique selon la revendication 7, caractérisé par le fait que les deux cadres sont du type monospire.

**FIG.1**

OSC 27,12 MHz (1)

AMPLIFICATEUR ACCORDÉ MODULABLE (2)

AMPLI DE PUISSANCE (4)

GÉNÉRATEUR DE MODULATION (3)

5

**FIG.2**

OSC 27,12 MHz (1)

AMPLIFICATEUR ACCORDÉ MODULABLE (2)

AMPLI DE PUISSANCE (4)

GÉNÉRATEUR DE MODULATION (3)

DEPHASEUR 90° (à 27,12 MHz) (6)

AMPLI DE PUISSANCE (7)

90°

5

8

| Catégorie | Citation du document avec indication, en cas de besoin, des parties pertinentes | Revendica-tion concernée |
|---|---|---|
| | **DOCUMENTS CONSIDERES COMME PERTINENTS** | |
| | FR - A - 818 881 (MATERIEL TELEPHONIQUE) <br> * Page 2, lignes 25-35; page 3, lignes 64-70 * | 1,2,7 |
| | -- | |
| | GB - A - 315 367 (LUEDKE) <br> Page 1, lignes 32-77 * | 1,2-4, 7 |
| | -- | |
| | DE - A - 2 716 672 (RODLER) <br> * Page 24, dernier alinéa; page 25, alinéa 1; figure 13 * | 1,3,8 |
| | -- | |
| | FR - A - 2 222 106 (KRAUS) <br> * Page 5, lignes 14-22; page 6, lignes 1-3; figure 4 * | 1,4,5 |
| | -- | |
| | FR - A - 1 239 590 (NEMEC) <br> * Page 2, colonne de gauche, alinéa 3, colonne de droite, lignes 1-8 et alinéa 3 * | 1,2-4, 7 |
| | -- | |
| | FR - A - 2 210 420 (NOGIER) <br> * Page 4, lignes 17-24; page 5, lignes 2,3; page 6, les 4 dernières lignes * | 1 |
| | -- | |
| | FR - A - 2 370 483 (CONSTANTINES-CU) <br> * Page 3, ligne 12 à page 4, ligne 10 * | 2,5,6 |
| | | ./. |

**CLASSEMENT DE LA DEMANDE (Int. Cl. ⁴)**

A 61 N 1/40

**DOMAINES TECHNIQUES RECHERCHES (Int. Cl. ³)**

A 61 N 1/40
1/42
1/32

**CATEGORIE DES DOCUMENTS CITES**

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons

&: membre de la même famille, document correspondant

X Le présent rapport de recherche a été établi pour toutes les revendications

| Lieu de la recherche | Date d'achèvement de la recherche | Examinateur |
|---|---|---|
| La Haye | 01-02-1980 | SIMON |

OEB Form 1503.1 06.78

| DOCUMENTS CONSIDERES COMME PERTINENTS | | | CLASSEMENT DE LA DEMANDE (Int. Cl. ³) |
|---|---|---|---|
| Catégorie | Citation du document avec indication, en cas de besoin, des parties pertinentes | Revendication concernée | |
| | DE - C - 956 790 (SANITAS)  *Page 2, lignes 13-28 *  --  ---- | 2,8 | |
| | | | **DOMAINES TECHNIQUES RECHERCHES (Int. Cl. ³)** |

OEB Form 1503.2   06.78